# EUROPEAN PATENT SPECIFICATION

(11) **EP 2 979 643 B1**
(45) Date of publication and mention of the grant of the patent: **25.10.2017**
(21) Application number: 14775344.6
(22) Date of filing: 28.01.2014
(51) Int. Cl.: A61B 8/00

(54) **ULTRASONIC IMAGING DEVICE AND ULTRASONIC IMAGE DISPLAY METHOD**
ULTRASCHALLBILDGEBUNGSVORRICHTUNG UND ULTRASCHALLBILDANZEIGEVERFAHREN
DISPOSITIF D'IMAGERIE ULTRASONORE ET PROCÉDÉ D'AFFICHAGE D'IMAGE ULTRASONORE

(30) Priority: 25.03.2013 JP 2013062535
(43) Date of publication of application: 03.02.2016
(73) Proprietor: Hitachi, Ltd., Chiyoda-ku Tokyo 100-8280 (JP)
(72) Inventor: NISHIURA, Tomofumi, Mitaka-shi Tokyo 181-8622 (JP)
(74) Representative: Strehl Schübel-Hopf & Partner
(86) International application number: PCT/JP2014/051735
(87) International publication number: WO 2014/156269

(56) References cited:
- JP-A- H05 192 336
- JP-A- H09 220 228
- JP-A- H10 262 963
- JP-A- 2006 223 712
- JP-A- 2007 252 725
- JP-A- 2012 019 873

## Description

### Technical Field

The present invention relates to an ultrasonic imaging apparatus and an ultrasound image display method, and in particular to an ultrasonic imaging apparatus and an ultrasound image display method that improve the image quality of an ultrasound image.

### Background Art

A conventional ultrasound image display method includes a step of specifying an ROI (region of interest) within a set of collected ultrasound data and a step of identifying a plurality of different image surfaces in the set of collected ultrasound data. This method further includes a step of deciding a significant edge from at least one boundary of the ROI on the basis of the plurality of image surfaces and a step of adjusting the ROI on the basis of the decided significant edge (for example, Patent Literature 1). JP 2007 252725 discloses a method and apparatus comprising ROI setting means by a user, determination means for determining foetal structure outside the ROI when the foetus moves and a volume rendering means for volume rendering of foetal data both within and outside the ROI to display the foetus.

### Citation List

### Patent Literature

Patent Literature 1: JP-A-2011-224362

### Summary of Invention

### Technical Problem

In a conventional ultrasound image display method, three-dimensional ultrasound image data suitable for calculation of an ROI may not be obtained because of movement of a probe, a body motion of an unborn baby and the like. If three-dimensional ultrasound image data unsuitable for calculation of an ROI is inputted, an inappropriate ROI is calculated, and an inappropriate three-dimensional ultrasound image is unexpectedly displayed. Therefore, the three-dimensional ultrasound image flickers, and the appearance of the image is bad. Especially in obstetrical diagnosis, since a diagnosing object, who is a mother, looks at a three-dimensional ultrasound image of an unborn baby, the diagnosing object is given an unfavorable impression when the appearance of the three-dimensional ultrasound image of the unborn baby is bad.

An object of the present invention is to provide an ultrasonic imaging apparatus and an ultrasound image display method that prevent a three-dimensional ultrasound image from flickering due to an inappropriate ROI being calculated, and improve the image quality of the ultrasound image.

### Solution to Problem

An ultrasonic imaging apparatus of the present invention is provided with: an ROI calculating section configured to calculate an ROI from ultrasound image data; a judgment section configured to judge whether the calculation of the ROI is successful or not on the basis of at least one of a position with a predetermined brightness difference and the number of positions with the brightness difference in the ultrasound image data; and a compensation section configured to compensate a failed ultrasound image based on the ROI judged to be failed by the judgment section with a successful ultrasound image based on the ROI judged to be successful by the judgment section.

### Advantageous Effects of Invention

According to the present invention, it is possible to prevent a three-dimensional ultrasound image from flickering due to an inappropriate ROI being calculated, and improve the image quality of the ultrasound image.

### Brief Description of Drawings

[Figure 1] Figure 1 is a block diagram showing an example of an ultrasonic imaging apparatus of the present embodiment.
[Figure 2] Figure 2 is a block diagram showing an example of an ultrasound image constructing section.
[Figure 3] Figure 3 is a block diagram showing an example of an image generating section.
[Figure 4] Figure 4 is a diagram illustrating precutting of three-dimensional ultrasound image data.
[Figure 5] Figure 5 is a diagram illustrating success/failure of ROI calculation.
[Figure 6] Figure 6 is a flowchart illustrating an operation of the ultrasonic imaging apparatus.
[Figure 7] Figure 7 is a diagram illustrating that, when ROI calculation fails, a failed ultrasound image is compensated with a successful ultrasound image.
[Figure 8] Figure 8 is a block diagram showing another example of the ultrasound image constructing section.
[Figure 9] Figure 9 is a diagram illustrating marks at the time of ROI calculation being successful/failed.
[Figure 10] Figure 10 is a diagram illustrating that, when ROI calculation fails, a failed ultrasound image is compensated with a successful ultrasound image, and a mark is given.
[Figure 11] Figure 11 is a diagram illustrating the kinds of marks displayed on a successful ultrasound image.

### Description of Embodiments

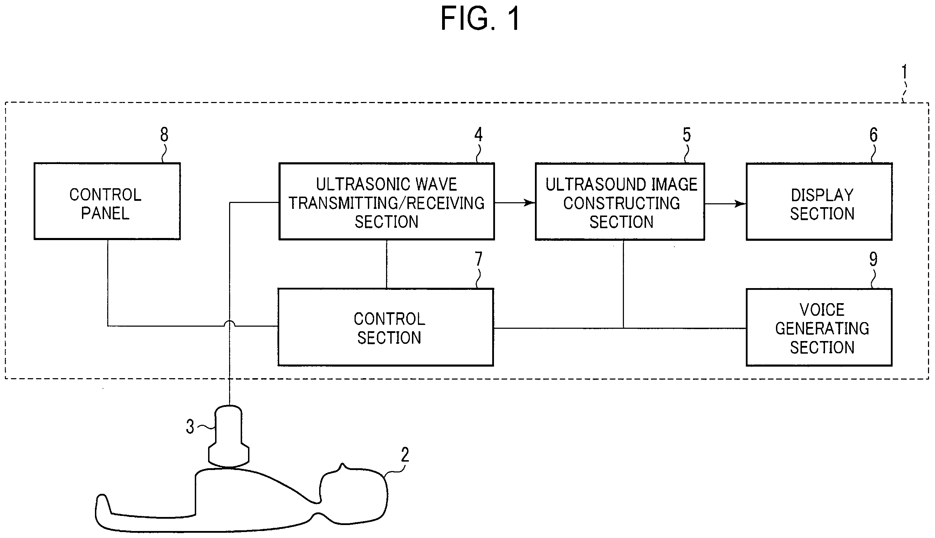

An ultrasonic imaging apparatus of an embodiment of the present invention will be described below with the use of drawings. Figure 1 is a block diagram showing an example of the ultrasonic imaging apparatus of the present embodiment. An ultrasonic imaging apparatus 1 may be used as an ultrasound diagnostic apparatus.

The ultrasonic imaging apparatus 1 transmits and receives an ultrasonic wave into a diagnosing object 2, and, using an obtained reflected echo signal, forms and displays a two-dimensional ultrasound image or a three-dimensional ultrasound image for a diagnosis region. The ultrasonic imaging apparatus 1 is provided with: an ultrasound probe 3 provided with transducer elements which radiate an ultrasonic wave to the diagnosing object 2 and receives the ultrasonic wave, an ultrasonic wave transmitting/receiving section 4 which transmits/receives an ultrasonic signal, an ultrasound image constructing section 5 which constructs a two-dimensional ultrasound image (a B-mode image) or a three-dimensional ultrasound image on the basis of a receive signal, a display section 6 which displays an ultrasound image constructed at the ultrasound image constructing section 5, a control section 7 which controls each component, a control panel 8 which gives an instruction to the control section 7, and a voice generating section 9 which generates a voice such as a warning.

On the ultrasound probe 3, the transducer elements corresponding to 1 to m channels are arranged in a major-axis direction of the ultrasound probe 3, and the transducer elements are cut in k in a minor-axis direction of the ultrasound probe 3 so that the transducer elements corresponding to 1 to k channels are arranged. By changing delay time given to each of the transducer elements in the minor-axis direction (1 to k channels), the ultrasound probe 3 can perform focusing of a transmit wave or a receive wave not only in the major-axis direction but also in the minor-axis direction and can acquire not only two-dimensional ultrasound image data but also three-dimensional ultrasound image data. By changing the amplitude of an ultrasonic transmit signal to be given to each transducer element in the minor-axis direction, the ultrasound probe 3 can perform weighting of a transmit wave. By changing the degree of amplification or the degree of attenuation of an ultrasonic receive signal from each transducer element in the minor-axis direction, the ultrasound probe 3 can perform weighting of a receive wave. The ultrasound probe 3 can perform aperture control by turning on/off each of the transducer elements in the minor-axis direction. Further, as a scanning system of the ultrasound probe 3, there are a sector scanning system, a linear scanning system, a convex scanning system, and a radial scanning system and the like. Further, the ultrasound probe 3 may acquire ultrasound image data by scanning an ultrasonic wave while mechanically causing the transducer elements to make a reciprocating movement in the minor-axis direction relative to the diagnosing object 2.

The ultrasonic wave transmitting/receiving section 4 supplies a transmit signal to the ultrasound probe 3 and processes a received reflected echo signal. The ultrasonic wave transmitting/receiving section 4 is provided with a wave transmitting circuit which controls the ultrasound probe 3 to perform wave transmission of an ultrasonic beam, a wave receiving circuit which receives a reflected echo signal of a reflected ultrasonic beam from inside the diagnosing object 2 to collect biological information, and a control circuit which controls these.

The ultrasound image constructing section 5 converts ultrasound image data (a reflected echo signal) processed by the ultrasonic wave transmitting/receiving section 4 to an ultrasound image (for example, an ultrasound cross-section region image or the like). Further, the ultrasound image constructing section 5 calculates an ROI (region of interest) from ultrasound image data, judges whether the ROI calculation is successful or not on the basis of at least one of a position with a predetermined brightness difference and the number of positions with the predetermined brightness difference in the ultrasound image data, and compensates a failed ultrasound image based on an ROI judged to be failed with a successful ultrasound image based on an ROI judged to be successful.

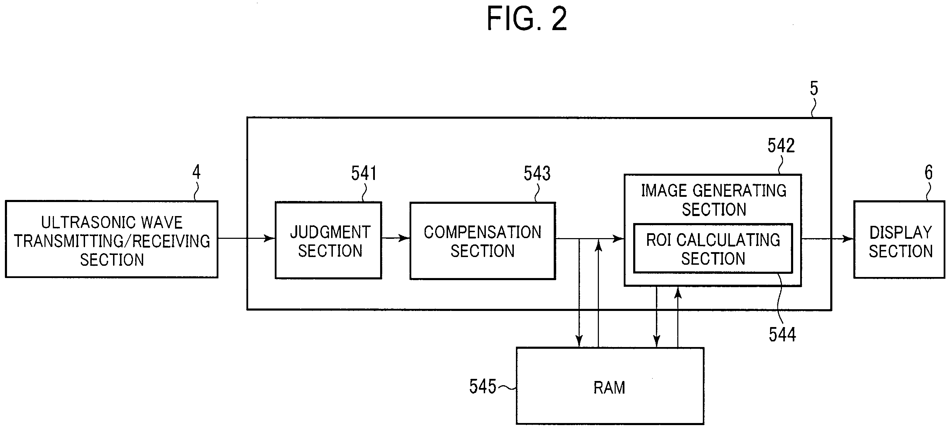

Figure 2 is a block diagram showing an example of the ultrasound image constructing section 5. As shown in Figure 2, the ultrasound image constructing section 5 is provided with: an image generating section 542 which calculates an ROI (region of interest) from ultrasound image data and images an ultrasound image and various kinds of Doppler images, a judgment section 541 which judges whether the ROI calculation is successful or not on the basis of at least one of a position with a predetermined brightness difference and the number of positions with the predetermined brightness difference in the ultrasound image data, and a compensation section 543 which compensates a failed ultrasound image based on an ROI judged to be failed by the judgment section 541 with a successful ultrasound image based on an ROI judged to be successful by the judgment section 541. Further, the ultrasound image constructing section 5 is provided with a storage section (a RAM 545) which stores ROI information (such as coordinates) about an ROI judged to be successful by the judgment section 541 and stores a successful ultrasound image based on the ROI judged to be successful by the judgment section 541. The image generating section 542 is provided with an ROI calculating section 544 which calculates an ROI from ultrasound image data.

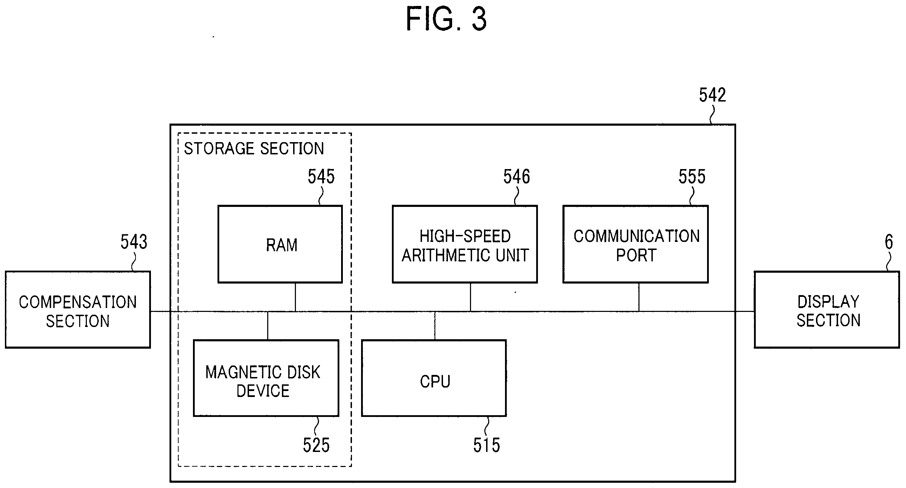

Figure 3 is a block diagram showing an example of the image generating section 542 (including the ROI calculating section 544). As shown in Figure 3, the image generating section 542 is provided with: a CPU (central processing unit) 515, a storage section including a magnetic disk device 525 and the RAM 545, a high-speed arithmetic unit 546 and a communication port 555. By performing signal processing of biological information received by the ultrasonic wave transmitting/receiving section 4, the image generating section 542 (the ROI calculating section 544) can calculate an ROI from ultrasound image data and image a two-dimensional ultrasound image, a three-dimensional ultrasound image and various kinds of Doppler images. Further, as shown in Figure 3, the image generating section 542 may be provided with the storage section (the magnetic disk device 525 and the RAM 545).

The display section 6 displays display information, such as an ultrasound image (for example, an ultrasound cross-section region image) generated by the ultrasound image constructing section 5, control information required for control by the control section 7 and user setting information, by control of a display control section (not shown). The display section 6 includes a CRT monitor, a liquid crystal monitor and the like. Further, the display control section is a display control system which includes a graphic processor and the like.

The voice generating section 9 generates a voice on the basis of an ultrasound image generated by the ultrasound image constructing section 5. The voice generating section 9 includes a speaker and the like.

The control section 7 controls an operation of each component. The control section 7 includes a computer system for control provided with an interface with a user interface circuit. The control section 7 controls the ultrasonic wave transmitting/receiving section 4 in accordance with a user interface and information from the user interface. Further, the control section 7 performs control such as transmitting biological information received by the ultrasonic wave transmitting/receiving section 4 to the ultrasound image constructing section 5 and transmitting information imaged by the ultrasound image constructing section 5 to the display control section.

The ultrasonic imaging apparatus 1 acquires a plurality of pieces of ultrasound image data (ultrasound cross-section region image data) of a diagnosis region of the diagnosing object 2 by electronically or mechanically swinging the ultrasound probe 3 in a direction orthogonal to a plane forming a rectangle or a fan shape, which transmits and receives an ultrasonic wave, and generates three-dimensional ultrasound image data using the plurality of pieces of ultrasound image data (ultrasound cross-section region image data). The ultrasonic imaging apparatus 1 constructs a three-dimensional ultrasound image from three-dimensional ultrasound image data by a method such as a voxel method and a volume rendering method and displays the three-dimensional ultrasound image on the display section 6 in real time.

Next, an operation of the ultrasonic imaging apparatus 1 will be described with the use of drawings. In the present embodiment, description will be made on an operation of the ultrasound image constructing section 5 calculating an ROI and generating a three-dimensional ultrasound image in real time.

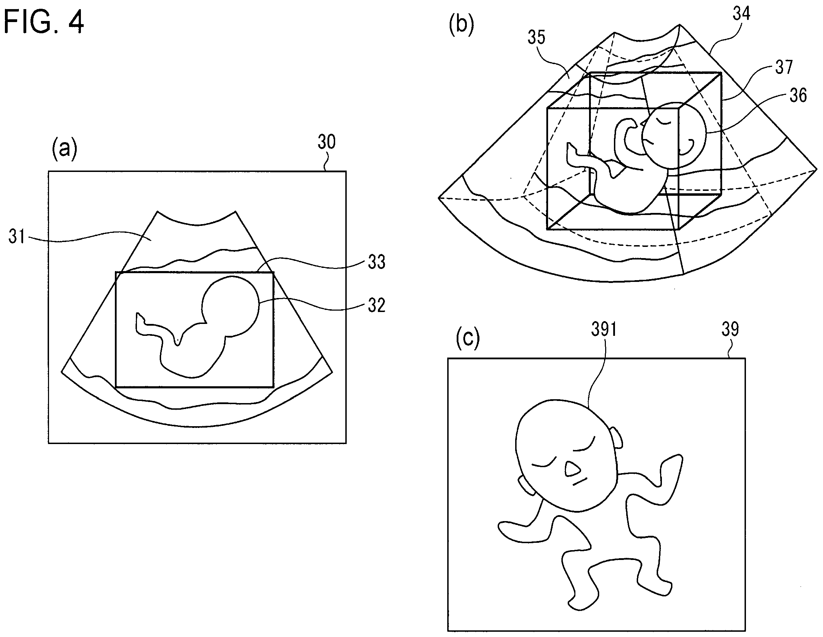

Figure 4 is a diagram illustrating precutting of three-dimensional ultrasound image data. Figure 4(a) is a diagram showing an ROI setting screen 30 for setting an ROI. In Figure 4(a), a state is shown in which the ROI calculating section 544 calculates and sets an ROI 33 in order to obtain three-dimensional ultrasound image data which does not include a placenta 31 but includes an unborn baby 32. The ROI calculating section 544 stores the coordinates of the boundary of the ROI 33 into the storage section and renders the boundary of the ROI 33 on the ROI setting screen 30. Figure 4(b) is a diagram conceptually illustrating a three-dimensional ultrasound image data process. As shown in Figure 4(b), three-dimensional ultrasound image data 34 constitutes a three-dimensional area. The ROI calculating section 544 sets a three-dimensional ROI 37 by three-dimensionally constructing the ROI 33. The three-dimensional ROI 37 is cut out from the three-dimensional ultrasound image data 34. The three-dimensional ultrasound image data 34 includes three-dimensional ultrasound image data 36 corresponding to the unborn baby and three-dimensional ultrasound image data 35 corresponding to the placenta. In the three-dimensional ROI 37 cut out from the three-dimensional ultrasound image data 34, however, the three-dimensional ultrasound image data 35 corresponding to the placenta is removed, and the three-dimensional ultrasound image data 36 corresponding to the unborn baby is extracted. The image generating section 542 calculates the three-dimensional ROI 37 from the ultrasound image data and generates a three-dimensional ultrasound image 39 from which the placenta has been removed and which includes a three-dimensional ultrasound image 391 of the unborn baby, by a method such as a voxel method and a volume rendering method as shown in Figure 4(c). The image generating section 542 may store the coordinates of the boundary of the ROI 33 into the storage section and render the boundary of the three-dimensional ROI 37 in the three-dimensional ultrasound image.

In order to enhance quickness of ultrasound diagnosis, the ultrasonic imaging apparatus 1 observes the diagnosing object 2 (for example, an unborn baby) while displaying a three-dimensional ultrasound image in real time. However, there may be a case where, due to movement of the ultrasound probe 3 or a body motion the unborn baby, the ROI calculating section 544 cannot obtain ultrasound image data (three-dimensional ultrasound image data) suitable for calculation of the three-dimensional ROI 37, and calculation of the three-dimensional ROI 37 fails. If ultrasound image data unsuitable for calculation of an ROI is inputted, an ROI within an inappropriate range is calculated, and an inappropriate three-dimensional ultrasound image is unexpectedly displayed. Therefore, the three-dimensional ultrasound image flickers, and the appearance of the image is bad. Further, in the case of obtaining an image of a complicated structure like hand and foot parts of an unborn baby or a structure in which body tissues interlace complicatedly, the boundary of an ROI cannot be decided, so that an ROI range is not calculated, and an inappropriate three-dimensional ultrasound image in which all the range is rendered is unexpectedly displayed. Therefore, the three-dimensional ultrasound image flickers, and the appearance of the image is bad.

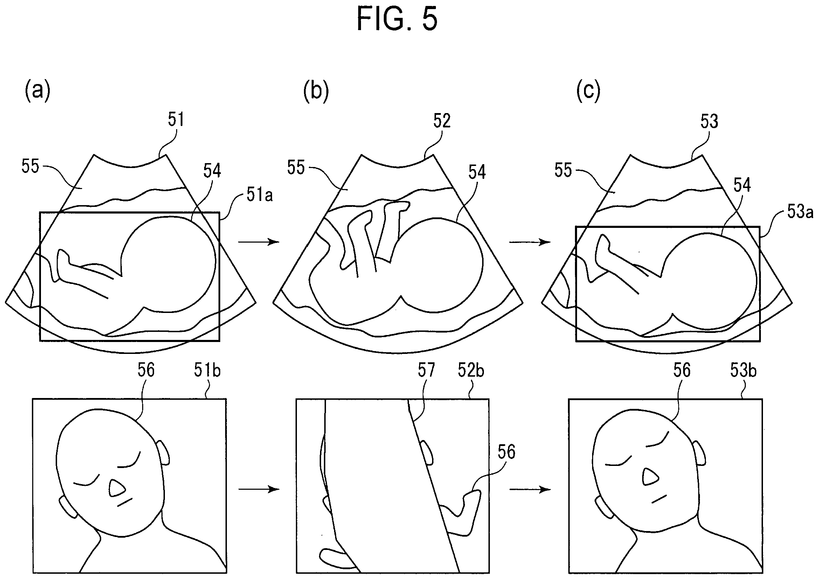

Figure 5 is a diagram illustrating success/failure of ROI calculation. In Figure 5, an unborn baby 54 (for example, the arms of the unborn baby 54) moves in time-series ultrasound images 51, 52 and 53. In the ultrasound images 51 and 53, by ROIs 51a and 53a judged to be successful by the judgment section 541 being acquired and three-dimensional ultrasound images 51b and 53b based on the ROIs 51a and 53a judged to be successful by the judgment section 541 being obtained, three-dimensional ultrasound image data corresponding to a placenta is removed, and three-dimensional ultrasound image data 56 corresponding to the unborn baby is extracted. On the other hand, in the ultrasound image 52, calculation of an ROI fails, and an appropriate ROI is not acquired. In this case, when the three-dimensional ultrasound image data 57 corresponding to the placenta 55 is included, and the three-dimensional ultrasound image data 56 corresponding to the unborn baby is extracted, a three-dimensional ultrasound image (a failed ultrasound image) 52b in which the three-dimensional ultrasound image data 57 corresponding to the placenta is included is unexpectedly displayed. As a result, when the three-dimensional ultrasound images 51b, 52b and 53b are displayed in a time series in real time, the three-dimensional ultrasound images flicker, and the appearance of the images is bad.

The ultrasonic imaging apparatus 1 of the present embodiment judges whether ROI calculation is successful or not on the basis of at least one of a position with a predetermined brightness difference and the number of positions with the predetermined brightness difference in ultrasound image data, and compensates an ultrasound image for which ROI calculation is judged to be failed with an ultrasound image for which ROI calculation is judged to be successful.

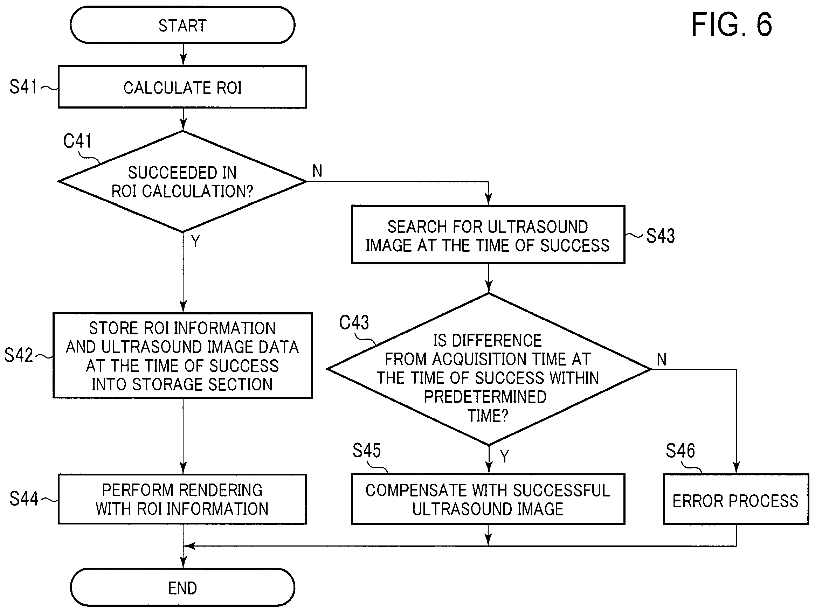

Figure 6 is a flowchart illustrating an operation of the ultrasonic imaging apparatus 1. As shown in Figure 6, the ROI calculating section 544 calculates an ROI from ultrasound image data at step S41. The ROI calculating section 544 calculates an ROI to be set for the three-dimensional ultrasound image data 34 in Figure 4. The ROI calculating section 544 can acquire a two-dimensional ultrasound image which is a section of the three-dimensional ultrasound image data 34 and causes the boundary between the placenta and amniotic fluid to be the boundary of the ROI by an edge extraction method.

The ROI calculating section 544 may cause the boundary between the amniotic fluid and the unborn baby to be the boundary of the ROI by the edge extraction method. Further, the ROI calculating section 544 may cause an intermediate position between the boundary between the placenta and the amniotic fluid and the boundary between the amniotic fluid and the unborn baby to be the boundary of the ROI. Further, the ROI calculating section 544 may divide the ultrasound image data (the two-dimensional ultrasound image data) into a plurality of blocks, classify the plurality of blocks into a block including the placenta, a block including the unborn baby and a block including the placenta and the unborn baby on the basis of brightness of each block, and cause the boundary of any of the classified blocks to be the boundary of the ROI (a two-dimensional ROI or a three-dimensional ROI). It is possible to perform the area division for the three-dimensional ultrasound image data 34 and decide the boundary of the three-dimensional ROI from a three-dimensional space. Furthermore, the ROI calculating section 544 may three-dimensionally construct an ROI from a plurality of two-dimensional ultrasound images by continuously arranging the boundaries of the ROIs of the two-dimensional ultrasound images (two-dimensional ROIs) along a predetermined coordinate axis to set the three-dimensional ROI 37. At this time, as for the boundary of the three-dimensional ROI, the boundaries of the ROIs of the two-dimensional ultrasound images (the two-dimensional ROIs) are continuously arranged along a coordinate axis orthogonal to a section of the two-dimensional ultrasound images, and, thereby, the three-dimensional ROI 37 can be set.

At step C41, the judgment section 541 judges whether the ROI calculation is successful or not on the basis of at least one of a position with a predetermined brightness difference and the number of positions with the predetermined brightness difference in the ultrasound image data. In the present embodiment, an ROI (region of interest) is calculated and set in order to remove a placenta and generate a three-dimensional ultrasound image of an unborn baby.

As shown in Figure 5(b), when the placenta with a high brightness and the unborn baby with a high brightness are close to each other, ROI calculation may fail in the edge extraction method because an edge indicating the boundary between the placenta and the unborn baby is not extracted, and the boundary of an ROI cannot be decided. Further, in the case of obtaining an image of a complicated structure like hand and foot parts of an unborn baby or a structure in which body tissues interlace complicatedly, ROI calculation may fail in the edge extraction method because a plurality of positions having a significant edge exist, and the boundary of an ROI cannot be decided. Further, even in the case of dividing the ultrasound image data into a plurality of blocks, the blocks cannot be appropriately classified into a block including the placenta, a block including the unborn baby and a block including the placenta and the unborn baby in a situation where the placenta and the unborn baby are close to each other, and the boundary of an ROI cannot be decided. Therefore, ROI calculation may fail.

The judgment section 541 judges whether the ROI calculation is successful or not on the basis of at least one of a position with a predetermined brightness difference and the number of positions with the predetermined brightness difference in the ultrasound image data. For example, whether the ROI calculation is successful or not is judged by whether the edge intensity of the boundary of the ROI exceeds a predetermined threshold or not. Further, the size or position (the coordinates) of the ROI is compared among a plurality of frames, and whether the ROI calculation is successful or not is judged by whether difference among the plurality of frames exceeds a predetermined threshold or not. Further, in the case where the boundary surface of the three-dimensional ROI is constructed in a three-dimensional space, angles (or slopes) of normal lines of a curved surface forming the boundary surface of the three-dimensional ROI are calculated on the curved surface, and whether the ROI calculation is successful or not is judged by whether the angle (or slope) of a normal line exceeding a predetermined threshold exists or not.

The judgment section 541 judges whether the ROI calculation is successful or not on the basis of at least one of: whether the number of positions with a predetermined bright difference in the ultrasound image data exceeds a predetermined threshold or not, whether the rate of the number of positions with the predetermined bright difference in the ultrasound image data exceeds a predetermined threshold or not, whether a statistic of the number of positions with the predetermined bright difference in the ultrasound image data exceeds a predetermined threshold or not, whether change in the coordinates of the ROI calculated on the basis of a position with the predetermined brightness difference in the ultrasound image data exceeds a predetermined threshold or not, whether change in the size of the ROI calculated on the basis of the position with the predetermined brightness difference in the ultrasound image data exceeds a predetermined threshold or not, whether change in the boundary of the ROI calculated on the basis of the position with the predetermined brightness difference in the ultrasound image data exceeds a predetermined threshold or not, whether the number of predetermined normal lines of the boundary of the ROI calculated on the basis of the position with the predetermined brightness difference in the ultrasound image data exceeds a predetermined threshold or not, whether the angle of a predetermined normal line of the boundary of the ROI calculated on the basis of the position with the predetermined brightness difference in the ultrasound image data exceeds a predetermined threshold or not, whether a statistic of the predetermined normal lines of the boundary of the ROI calculated on the basis of the position with the predetermined brightness difference in the ultrasound image data exceeds a predetermined threshold or not, and whether a value of correlation with the ROI of a successful ultrasound image exceeds a predetermined threshold or not.

For example, whether the ROI calculation is successful or not is judged by whether or not the number of positions with a predetermined brightness difference (for example, significant edges extracted by the edge extraction method) exceeds a predetermined threshold along a predetermined coordinate axis of an ultrasound image. Further, the number of positions with a predetermined brightness difference (for example, positions having a brightness difference exceeding a threshold and positions having a brightness difference equal to or below the threshold) is counted along a predetermined coordinate axis of the ultrasound image, and whether the ROI calculation is successful or not is judged by whether the rate of the number of the position having a brightness difference exceeding the threshold exceeds a predetermined or not. Further, whether the ROI calculation is successful or not is judged by whether or not a statistic of the number of positions with the predetermined bright difference (for example, the average value, median, deviation and variance of the number of the positions having a brightness difference exceeding a threshold, a value of correlation with the number of positions with the predetermined brightness difference in a successful ultrasound image, and the like) exceeds a predetermined threshold along a predetermined coordinate axis of the ultrasound image.

Whether the ROI calculation is successful or not is judged by whether change (temporal change or spatial change) in the coordinates of the ROI (the coordinates of the boundary or the center of gravity, and the like) exceeds a predetermined threshold or not. For example, if the center of gravity of the ROI moves (temporal change) exceeding a threshold, the ROI calculation is judged to be failed. Further, if a spatial differentiation value of the boundary of the ROI exceeds a threshold, the ROI calculation is judged to be failed.

Whether the ROI calculation is successful or not is judged by whether change (temporal change or spatial change) in the size of the ROI (the length, area, volume and the like) exceeds a predetermined threshold or not. For example, the area of the ROI in a predetermined time increases to be larger than a threshold (temporal change), the ROI calculation is judged to be failed. Further, if a spatial differentiation value of the length of the boundary of the ROI along a predetermined coordinate axis exceeds a threshold, the ROI calculation is judged to be failed.

Whether the ROI calculation is successful or not is judged by whether change (temporal change or spatial change) in the boundary (the boundary line, boundary surface or the like) of the ROI exceeds a predetermined threshold or not. For example, when the boundary surface of the three-dimensional ROI in a predetermined time moves, exceeding a threshold (temporal change), the ROI calculation is judged to be failed. Further, if a spatial differentiation value of the boundary surface of the three-dimensional ROI exceeds a threshold, the ROI calculation is judged to be failed.

Whether the ROI calculation is successful or not is judged by whether the number of predetermined normal lines (for example, normal lines having a predetermined angle) of the boundary of the ROI exceeds a predetermined threshold or not. Further, whether the ROI calculation is successful or not is judged by whether the angle of a predetermined normal line of the boundary of the ROI exceeds a predetermined threshold or not. For example, if the angle of a normal line of the boundary surface (the angle of a normal line is related to the inclination of a boundary surface) of the three-dimensional ROI exceeds a threshold, the ROI calculation is judged to be failed. Further, whether the ROI calculation is successful or not is judged by whether a statistic of the predetermined normal lines of the boundary of the ROI exceeds a predetermined threshold or not. For example, if at least one of the average value, median, deviation and variance of the angles of normal lines of the boundary surface of the three-dimensional ROI exceeds a threshold, the ROI calculation is judged to be failed.

Whether the ROI calculation is successful or not is judged by whether a value of correlation with the ROI of a successful ultrasound image exceeds a predetermined threshold or not. For example, when ROI information about a successful ultrasound image acquired in the past is read out from the storage section (the magnetic disk device 525 or the RAM 545), and a value of correlation with the ROI of the successful ultrasound image based on the ROI information exceeds a predetermined threshold, the ROI calculation is judged to be successful.

In the case of dividing the ultrasound image data into a plurality of blocks, the judgment section 541 may judge whether the ROI calculation is successful or not on the basis of at least one of a block having a predetermined brightness difference and the number of blocks having the predetermined brightness difference in the ultrasound image data. For example, if at least one of the average value, median, deviation, variance and rate of the number of blocks having the predetermined brightness difference exceeds a threshold, the ROI calculation is judged to be failed.

If the judgment section 541 judges at step C41 in Figure 6 that the ROI calculation is successful, the process proceeds to step S42, where the ROI calculating section 544 stores ROI information (the coordinates of the position and boundary of the ROI and the like) and the ultrasound image data at the time of the ROI calculation being judged to be successful by the judgment section 541 into the storage section. In this case, the ROI calculating section 544 stores the ROI information (the time of acquiring or recording the ROI, and the like) at the time of the ROI calculation being judged to be successful into the storage section (the RAM 545) in association with the ultrasound image data. The ROI information includes information showing success of the ROI calculation, a part or all of a group of coordinates forming the ROI (including a part or all of a group of coordinates forming the boundary surface of the three-dimensional ROI on a three-dimensional space), and a geometrical formula of a figure forming the ROI (including a geometrical formula forming the boundary surface of the three-dimensional ROI), and the like. Further, the ROI information includes the time when the ROI was calculated (ROI acquisition time) and the time of recording the ROI. Furthermore, the ROI information may include the ID of the three-dimensional ultrasound image data used for the ROI calculation, the ID of a diagnosis chart, and a part or all of information included in the diagnosis chart. Further, the ROI calculating section 544 may store ROI information about a plurality of ROIs for which ROI calculation has been judged to be successful, into the storage section (the RAM 545).

Then, at step S44, the image generating section 542 generates an ultrasound image in the ROI. For example, as shown in Figure 4(c), the image generating section 542 generates the three-dimensional ultrasound image 39 from which the placenta has been removed and which includes the three-dimensional ultrasound image 391 of the unborn baby. The image generating section 542 stores the successful ultrasound image based on the ROI judged to be successful by the judgment section 541 and the acquisition time (or recording time) of the ultrasound image into the storage section (the RAM 545) in association with the ROI information. Further, the image generating section 542 may store a plurality of ultrasound images corresponding to a plurality of ROIs judged to be successful by the judgment section 541, into the storage section (the RAM 545). Thus, the storage section (the RAM 545) stores successful ultrasound images generated from ultrasound image data at the time of having succeeded in ROI calculation.

On the other hand, if the judgment section 541 judges at step C41 in Figure 6 that the ROI calculation is failed, the process proceeds to step S43, where the compensation section 543 searches for a successful ultrasound image based on an ROI judged to be successful by the judgment section 541. In order to compensate an ultrasound image based on the ROI judged to be failed by the judgment section 541 (a failed ultrasound image) with an ultrasound image based on an ROI judged to be successful by the judgment section 541 (a successful ultrasound image), the compensation section 543 searches the storage section (the RAM 545) for the ultrasonic by ROI information (acquisition time and the like). For example, when the compensation section 543 performs the search, the compensation section 543 judges whether the failed ultrasound image can be compensated with a successful ultrasound image or not by whether difference between the acquisition time of the failed ultrasound image and the acquisition of the successful ultrasound image is within a predetermined time or not at step C43. This is because, if the difference between the acquisition time of the failed ultrasound image and the acquisition time of the successful ultrasound image exceeds the predetermined time, there is a possibility that, when the failed ultrasound image is compensated with the successful ultrasound image, a three-dimensional ultrasound image giving an uncomfortable feeling is displayed though flicker of the three-dimensional ultrasound image can be prevented. That is, if the difference between the acquisition time of the failed ultrasound image and the acquisition time of the successful ultrasound image is within the predetermined time, the compensation section 543 judges that the failed ultrasound image can be compensated with the successful ultrasound image. Further, the compensation section 543 judges whether the failed ultrasound image can be compensated with the successful ultrasound image or not by checking whether the ID of the diagnosis chart corresponds or not. That is, if the ID of the diagnosis chart corresponds, the compensation section 543 judges that the failed ultrasound image can be compensated with the successful ultrasound image.

If the search by the compensation section 543 is successful at step C43, the process proceeds to step S45, where the compensation section 543 compensates the ultrasound image based on the ROI judged to be failed by the judgment section 541 (the failed ultrasound image) with the ultrasound image based on the ROI judged to be successful by the judgment section 541 (the successful ultrasound image). Specifically, the compensation section 543 outputs a compensation instruction, and, in accordance with the compensation instruction, the image generating section 542 reads out the successful ultrasound image (including a successful ultrasound image obtained in the immediately previous frame) from the storage section (the RAM 545) on the basis of the position and acquisition time of the failed ultrasound image and replaces the failed ultrasound image with the successful ultrasound image. Thus, the compensation section 543 compensates the failed ultrasound image based on the ROI judged to be failed by the judgment section 541 with a successful ultrasound image generated from ultrasound image data at the time of having succeeded in ROI calculation.

Further, at steps S43 and C43, the compensation section 543 may search for ROI information judged to be successful by the judgment section 541 (successful ROI information). In this case, at step S45, in order to compensate the ultrasound image based on the ROI judged to be failed by the judgment section 541 (a failed ROI) (a failed ultrasound image) with the ultrasound image based on the ROI judged to be successful by the judgment section 541 (a successful ROI) (a successful ultrasound image), the compensation section 543 may output a compensation instruction to generate an ultrasound image in the successful ROI to the image generating section 542 on the basis of the boundary of the retrieved successful ROI, by a method such as a voxel method and a volume rendering method (for example, a ray casting method). Thus, a three-dimensional ultrasound image (for example, an ultrasound image of an unborn baby) may be generated in real time with three-dimensional ultrasonic data surrounded by the successful ROI as a rendering target, on the basis of the ROI information judged to be successful to be used for compensation of a failed ultrasound image as a successful ultrasound image. That is, the compensation section 543 may compensate the failed ultrasound image based on the ROI judged to be failed by the judgment section 541 with a successful ultrasound image generated in real time from ultrasound image data on the basis of the boundary of an ROI at the time of having succeeded in ROI calculation.

If the search by the compensation section 543 fails at step C43, the process proceeds to step S46, where the compensation section 543 transmits an error signal to perform an error process. In the error process, the failed ultrasound image may be displayed as it is or may be forcedly compensated with the latest successful ultrasound image on a time axis. Otherwise, an error image (for example, a black image) may be displayed.

Thus, if it is judged by the judgment section 541 that the ROI calculation is successful, an ultrasound image is created by successful ROI information. On the other hand, if it is judged by the judgment section 541 that the ROI calculation is failed, it is judged whether there is a substitutable successful ultrasound image (or a successful ROI) or not (step C43), and then the failed ultrasound image is replaced (compensated) with the successful ultrasound image.

The successful ultrasound image used for compensation may be one successful ultrasound image or may be a successful ultrasound image obtained by performing averaging or weighted averaging of a plurality of successful ultrasound images. Further, the compensation section 543 may judge whether a part or all of the plurality of successful ultrasound images satisfy the judgment condition of step C43 or not, and, if a part or all of the plurality of successful ultrasound images satisfy the judgment condition of step C43, use the successful ultrasound image obtained by performing averaging or weighted averaging of the plurality of successful ultrasound images for compensation of the failed ultrasound image. Further, in the case where an ultrasound image is generated in real time on the basis of the boundary of a successful ROI, a real-time ultrasound image based on the boundary of a successful ROI obtained by performing averaging or weighted averaging of a plurality of pieces of successful ROI information (such as the coordinates of boundaries) which satisfy the judgment condition of step C43 may be used for compensation of the failed ultrasound image as a successful ultrasound image.

Further, the compensation section 543 may use the latest successful ultrasound image (or successful ROI) on a time axis for compensation of the failed ultrasound image. Further, the judgment section 541 may divide the ultrasound image data (two-dimensional ultrasound image data) into a plurality of blocks and judge whether ROI calculation is successful or not for each block, and the compensation section 543 may compensate a block for which ROI calculation is judged to be failed by the judgment section 541 with a block for which ROI calculation is judged to be successful by the judgment section 541.

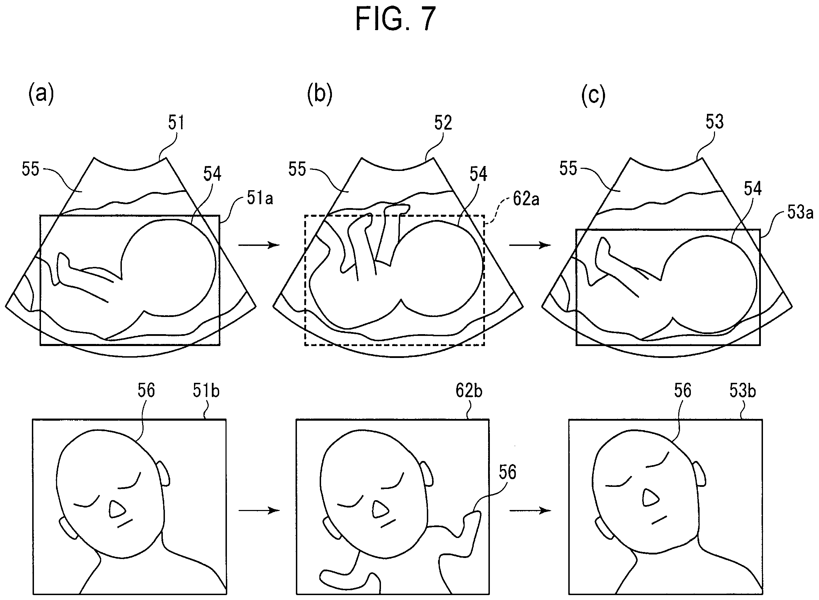

Figure 7 is a diagram illustrating that, when ROI calculation fails, a failed ultrasound image is compensated with a successful ultrasound image. In Figure 7, the unborn baby 54 (for example, the arms of the unborn baby 54) moves in the time-series ultrasound images 51, 52 and 53. Similarly to Figure 5, ROI calculation is successful in the ultrasound images 51 and 53, and the ROIs 51a and 53a are acquired (steps S41 and C41). ROI information about the ROIs 51a and 53a (the coordinates of the positions and boundaries of the ROIs and the like) and ultrasound image data are stored into the storage section (step S42). By the three-dimensional ultrasound images 51b and 53b being obtained, the three-dimensional ultrasound image data corresponding to the placenta is removed, and the three-dimensional ultrasound image data 56 corresponding to the unborn baby is extracted (step S44).

On the other hand, in the ultrasound image 52, ROI calculation fails, and an ROI is not acquired (steps S41 and C41). In this case, the compensation section 543 searches the storage section (the RAM 545) for a successful ultrasound image. In Figure 7, the latest successful ultrasound image is searched for on a time axis. Therefore, the compensation section 543 searches for the three-dimensional ultrasound image 51b of the ROI 51a as a successful ultrasound image. If the search by the compensation section 543 is successful (step C43), the compensation section 543 compensates the failed ultrasound image (the 52b in Figure 5) with a successful ultrasound image 62b (step S45). Alternatively, the successful ultrasound image 62b is generated in real time on the basis of the boundary of a successful ROI 62a corresponding to the ROI 51a judged to be successful by the judgment section 541, and the compensation section 543 compensates the failed ultrasound image (52b in Figure 5) with the successful ultrasound image 62b (step S45).

An embodiment according to the present invention has been described above. The present invention, however, is not limited thereto and can be changed/modified within a range described in claims.

For example, in the case where the ROI calculating section 544 sets a three-dimensional ROI by continuously arranging the boundaries of two-dimensional ROIs of two-dimensional ultrasound images along a predetermined coordinate axis, the judgment section 541 may judge whether calculation of the two-dimensional ROIs is successful or not, and the compensation section 543 may compensate a failed ultrasound image with a successful ultrasound image generated from ultrasound image data at the time of having succeeded in calculation of a two-dimensional ROI. Further, in the case where the ROI calculating section 544 sets a three-dimensional ROI by continuously arranging the boundaries of two-dimensional ROIs of two-dimensional ultrasound images along a predetermined coordinate axis, the judgment section 541 may judge whether calculation of the two-dimensional ROIs is successful or not, and the compensation section 543 may compensate a failed ultrasound image (a two-dimensional failed ultrasound image) with a successful ultrasound image (a two-dimensional successful ultrasound image) generated in real time from ultrasound image data on the basis of the boundary of a two-dimensional ROI at the time of having succeeded in calculation of the two-dimensional ROI. Thus, when a three-dimensional ROI is set, the compensation section 543 may compensate a two-dimensional failed ultrasound image with a two-dimensional successful ultrasound image for each of two-dimensional ROIs constituting the three-dimensional ROI (which are sections of the three-dimensional ROI).

Further, the ROI calculating section 544 may calculate a three-dimensional ROI from ultrasound image data; the judgment section 541 may judge whether the calculation of the three-dimensional ROI is successful or not; and the compensation section 543 may compensate a three-dimensional failed ultrasound image based on the three-dimensional ROI (a three-dimensional failed ultrasound image) with a three-dimensional successful ultrasound image based on a three-dimensional ROI (a three-dimensional successful ultrasound image). Thus, when a three-dimensional ROI is set, the compensation section 543 may compensate a three-dimensional failed ultrasound image with a three-dimensional successful ultrasound image for each three-dimensional ROI.

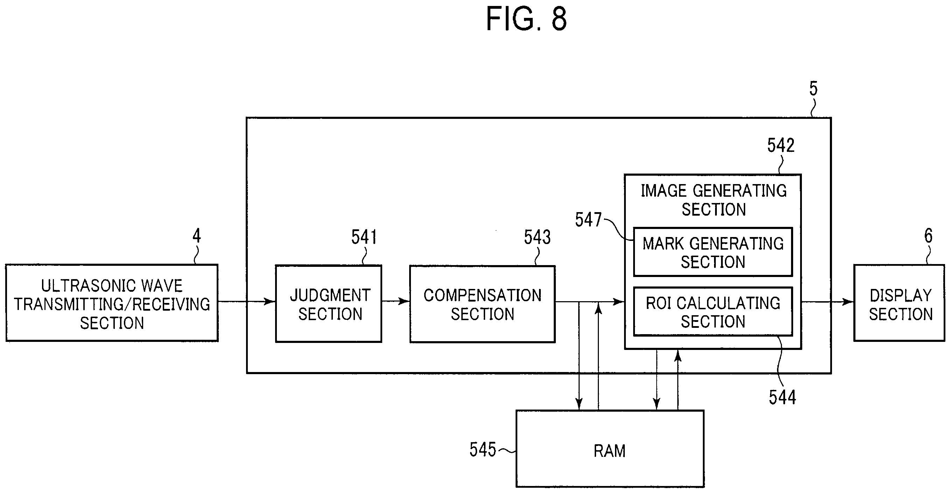

Further, as shown in Figure 8, the ultrasonic imaging apparatus 1 of the present embodiment may be provided with a mark generating section 547 which causes a mark showing whether ROI calculation is successful or not to be displayed on a successful ultrasound image.

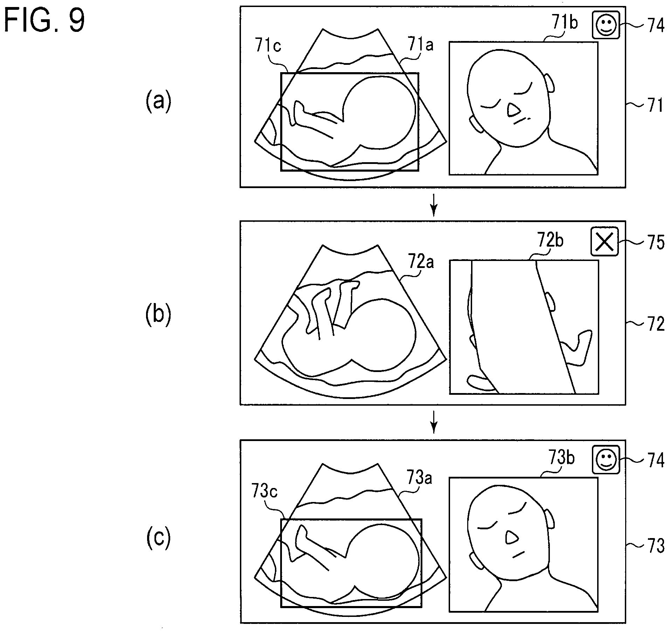

Figure 9 is a diagram illustrating marks at the time when ROI calculation is successful/failed. In ultrasound images 71a and 73a of display images 71 and 73, ROIs 71c and 73c judged to be successful by the judgment section 541 are acquired, three-dimensional ultrasound image 71b and 73b based on the ROIs 71c and 73c judged to be successful by the judgment section 541 are obtained, and the mark generating section 547 causes a success mark 74 showing that ROI calculation is successful to be displayed on the display section 6 until the frame switches to the next frame. On the other hand, in an ultrasound image 72a of a display image 72, calculation of an ROI fails, and an appropriate ROI is not acquired. A three-dimensional ultrasound image 72b based on an inappropriate ROI is obtained, and the mark generating section 547 causes a failure mark 75 showing failure of calculation of the ROI to be displayed on the display section 6 until the frame switches to the next switch. Alternatively, when the compensation section 543 performs an error process at step S46 in Figure 6, the mark generating section 547 causes the failure mark 75 to be displayed on the failed ultrasound image or an error image.

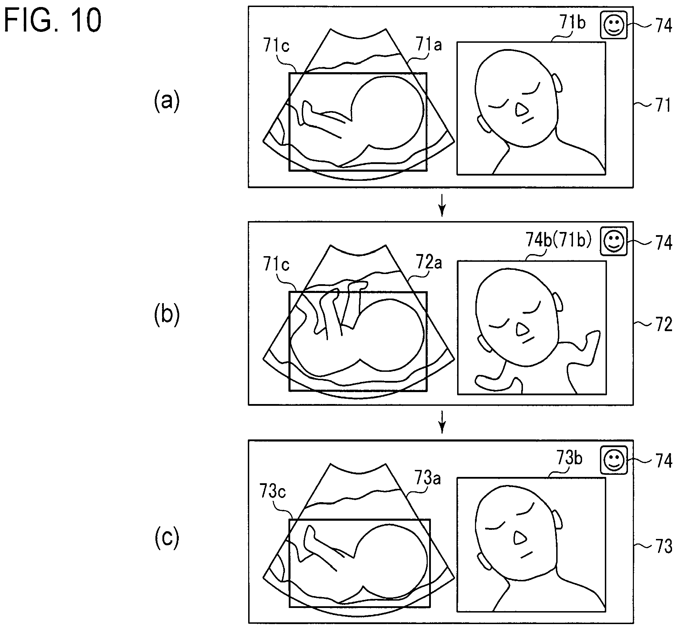

In the present embodiment, as shown in Figure 10, the compensation section 543 compensates a failed ultrasound image with a successful ultrasound image when ROI calculation fails, and, therefore, in the ultrasound image 72a of the display image 72, the compensation section 543 compensates a failed ultrasound image (72b in Figure 9) with a successful ultrasound image 74b (for example, the successful ultrasound image 71b obtained in the immediately previous frame). Alternatively, the successful ultrasound image 74b is generated in real time on the basis of the boundary of a successful ROI corresponding to the ROI 71c judged to be successful by the judgment section 541, and the compensation section 543 compensates the failed ultrasound image (72b in Figure 9) with the successful ultrasound image 74b. In this case, the mark generating section 547 causes the success mark 74 showing success of calculation of the ROI to be displayed on the successful ultrasound image.

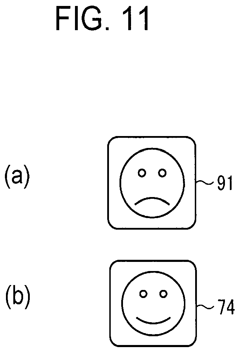

Further, the mark generating section 547 may cause a mark different from other successful ultrasound images to be displayed on a successful ultrasound image with which a failed ultrasound image is compensated. For example, a compensation mark 91 as shown in Figure 11(a) is caused to be displayed on a successful ultrasound image (the display image 72 in Figure 10) with which a failed ultrasound image is compensated, and the success mark 74 as shown in Figure 11(b) is caused to be displayed on ordinary successful ultrasound images (the display images 71 and 73 in Figure 10).

Further, the display section 6 may display whether ROI calculation is successful or not on a successful ultrasound image. For example, the display section 6 may display an ROI, and, by changing at least one of the color of the ROI, the boundary (a solid line, a dotted line or the like) of the ROI, the thickness of the boundary of the ROI, and the mark, display whether ROI calculation is successful or not on a successful ultrasound image. Further, the voice generating section 9 may generate a voice (for example, an alarm) showing whether ROI calculation is successful or not. Thus, by changing an image (such as a mark), a character string, a voice, vibration, and an ROI, whether calculation of the ROI is successful or not is shown.

Further, though the ultrasonic imaging apparatus 1 of the present embodiment compensates a failed ultrasound image with a successful ultrasound image for an ultrasound image of an unborn baby in a obstetrical division, a failed ultrasound image may be compensated with a successful ultrasound image for an ultrasound image of the liver, liver cell, liver blood vessel, gallbladder, bile duct, spleen, pancreas, kidney, adrenal, womb, ovary, prostate, stomach, intestine, vermiform, heart, blood vessel including artery and vein, thyroid, parathyroid, carotid artery, jugular vein, mammary, lymph node, digestive organ, womb, ovary, ureter, urinary bladder, cellular tissue and muscular tissue in addition to an unborn baby.

### Industrial Applicability

An ultrasonic imaging apparatus and an ultrasound image display method according to the present invention are useful as an ultrasonic imaging apparatus and an ultrasound image display method which is capable of preventing a three-dimensional ultrasound image from flickering due to an inappropriate ROI being calculated and improving the image quality of the ultrasound image and which improves the image quality of an ultrasound image.

### Reference Signs List

- 1: ultrasonic imaging apparatus
- 3: ultrasound probe
- 4: ultrasonic wave transmitting/receiving section
- 5: ultrasound image constructing section
- 8: control panel
- 9: voice generating section
- 525: magnetic disk device
- 541: judgment section
- 542: image generating section
- 543: compensation section
- 544: ROI calculating section
- 545: RAM
- 546: high-speed arithmetic unit
- 547: mark generating section
- 555: communication port

## Claims

1. An ultrasonic imaging apparatus comprising:
a ROI calculating section configured to calculate a ROI from ultrasound image data;
a judgment section configured to judge whether the calculation of the ROI is successful or not on the basis of at least one of a position with a predetermined brightness difference and the number of positions with the brightness difference in the ultrasound image data; and
a compensation section configured to compensate a failed ultrasound image based on the ROI judged to be failed by the judgment section with a successful ultrasound image based on the ROI judged to be successful by the judgment section.

2. The ultrasonic imaging apparatus according to claim 1, wherein the compensation section compensates the failed ultrasound image with the successful ultrasound image generated from the ultrasound image data at the time of having succeeded in the calculation of the ROI.

3. The ultrasonic imaging apparatus according to claim 1, wherein the compensation section compensates the failed ultrasound image with the successful ultrasound image generated in real time from ultrasound image data on the basis of a boundary of the ROI at the time of having succeeded in the calculation of the ROI.

4. The ultrasonic imaging apparatus according to claim 1, wherein the judgment section judges whether the calculation of the ROI is successful or not on the basis of at least one of whether the number of the positions with the brightness difference exceeds a predetermined threshold or not, whether a rate of the number of the positions with the brightness difference exceeds a predetermined threshold or not, whether a statistic of the number of the positions with the brightness difference exceeds a predetermined threshold or not, whether change in coordinates of the ROI calculated on the basis of the position with the brightness difference exceeds a predetermined threshold or not, whether change in a size of the ROI calculated on the basis of the position with the brightness difference exceeds a predetermined threshold or not, whether change in a boundary of the ROI calculated on the basis of the position with the brightness difference exceeds a predetermined threshold or not, whether the number of predetermined normal lines of the boundary of the ROI calculated on the basis of the position with the brightness difference exceeds a predetermined threshold or not, whether an angle of a predetermined normal line of the boundary of the ROI calculated on the basis of the position with the brightness difference exceeds a predetermined threshold or not, whether a statistic of the predetermined normal lines of the boundary of the ROI calculated on the basis of the position with the brightness difference exceeds a predetermined threshold or not, and whether a value of correlation with the ROI of the successful ultrasound image exceeds a predetermined threshold or not.

5. The ultrasonic imaging apparatus according to claim 1, wherein the compensation section judges whether the failed ultrasound image can be compensated with the successful ultrasound image on the basis of at least one of whether difference between acquisition time of the failed ultrasound image and acquisition time of the successful ultrasound image is within a predetermined time or not and whether an ID of a diagnosis chart corresponds or not.

6. The ultrasonic imaging apparatus according to claim 1, wherein
the ROI calculating section sets a three-dimensional ROI by continuously arranging boundaries of two-dimensional ROIs of two-dimensional ultrasound images along a predetermined coordinate axis;
the judgment section judges whether calculation of the two-dimensional ROIs is successful or not; and
the compensation section compensates the failed ultrasound image with the successful ultrasound image generated from the ultrasound image data at the time of having succeeded in the calculation of the two-dimensional ROI.

7. The ultrasonic imaging apparatus according to claim 1, wherein
the ROI calculating section sets a three-dimensional ROI by continuously arranging boundaries of two-dimensional ROIs of two-dimensional ultrasound images along a predetermined coordinate axis;
the judgment section judges whether calculation of the two-dimensional ROIs is successful or not; and
the compensation section compensates the failed ultrasound image with the successful ultrasound image generated in real time from ultrasound image data on the basis of a boundary of the two-dimensional ROI at the time of having succeeded in the calculation of the two-dimensional ROI.

8. The ultrasonic imaging apparatus according to claim 1, wherein
the ROI calculating section calculates a three-dimensional ROI from the ultrasound image data;
the judgment section judges whether the calculation of the three-dimensional ROI is successful or not; and
the compensation section compensates the failed ultrasound image, which is three-dimensional, based on the three-dimensional ROI with the successful ultrasound image, which is three-dimensional, based on the three-dimensional ROI.

9. The ultrasonic imaging apparatus according to claim 1, wherein the ROI calculating section divides the ultrasound image data into a plurality of blocks, classifies the plurality of blocks on the basis of brightness of each block, and calculates a boundary of the classified block as a three-dimensional ROI.

10. The ultrasonic imaging apparatus according to claim 1, wherein the judgment section divides the ultrasound image data into a plurality of blocks and judges whether the calculation of the ROI is successful or not on the basis of at least one of a position of the block having a predetermined brightness difference and the number of the blocks having the brightness difference.

11. The ultrasonic imaging apparatus according to claim 1, comprising a mark generating section configured to cause a mark showing whether the calculation of the ROI is successful or not to be displayed on the successful ultrasound image.

12. The ultrasonic imaging apparatus according to claim 11, wherein the mark generating section causes a mark different from the other successful ultrasound images to be displayed on the successful ultrasound image with which the failed ultrasound image is compensated.

13. The ultrasonic imaging apparatus according to claim 1, comprising a display section configured to display whether the calculation of the ROI is successful or not on the successful ultrasound image.

14. The ultrasonic imaging apparatus according to claim 1, comprising a voice generating section configured to generate a voice showing whether the calculation of the ROI calculation is successful or not.

15. An ultrasound image display method comprising:
calculating an ROI from ultrasound image data;
judging whether the calculation of the ROI is successful or not on the basis of at least one of a position with a predetermined brightness difference and the number of positions with the brightness difference in the ultrasound image data; and
compensating a failed ultrasound image based on the ROI judged to be failed with a successful ultrasound image based on the ROI judged to be successful.

## Patentansprüche

1. Ultraschallbildgebungsgerät mit
einem ROI-Berechnungsabschnitt, der dazu konfiguriert ist, aus Ultraschallbilddaten eine ROI zu berechnen,
einem Beurteilungsabschnitt, der dazu konfiguriert ist, basierend auf einer Position mit einer vorbestimmten Helligkeitsdifferenz und/oder der Anzahl von Positionen in den Ultraschallbilddaten mit der Helligkeitsdifferenz zu beurteilen, ob die Berechnung der ROI erfolgreich ist oder nicht, und
einem Kompensationsabschnitt, der dazu konfiguriert ist, ein basierend auf der durch den Beurteilungsabschnitt als gescheitert beurteilten ROI gescheitertes Ultraschallbild mit einem basierend auf der durch den Beurteilungsabschnitt als erfolgreich beurteilten ROI erfolgreichen Ultraschallbild zu kompensieren.

2. Ultraschallbildgebungsgerät nach Anspruch 1, wobei der Kompensationsabschnitt das gescheiterte Ultraschallbild mit dem erfolgreichen Ultraschallbild kompensiert, das aus den Ultraschallbilddaten zu dem Zeitpunkt erzeugt wurde, zu dem die Berechnung der ROI erfolgreich war.

3. Ultraschallbildgebungsgerät nach Anspruch 1, wobei der Kompensationsabschnitt das gescheiterte Ultraschallbild mit dem erfolgreichen Ultraschallbild kompensiert, das in Echtzeit aus den Ultraschallbilddaten basierend auf einer Grenze der ROI zu dem Zeitpunkt erzeugt wurde, zu dem die Berechnung der ROI erfolgreich war.

4. Ultraschallbildgebungsgerät nach Anspruch 1, wobei der Beurteilungsabschnitt auf der Grundlage, ob die Anzahl der Positionen mit der Helligkeitsdifferenz einen vorbestimmten Schwellenwert überschreitet, ob eine Rate der Anzahl der Positionen mit der Helligkeitsdifferenz einen vorbestimmten Schwellenwert überschreitet, ob eine Statistik der Anzahl der Positionen mit der Helligkeitsdifferenz einen vorbestimmten Schwellenwert überschreitet, ob eine Änderung in Koordinaten der ROI, die basierend auf der Position mit der Helligkeitsdifferenz berechnet wurde, einen vorbestimmten Schwellenwert überschreitet, ob eine Änderung in einer Größe der ROI, die auf Basis der Position mit der Helligkeitsdifferenz berechnet wurde, einen vorbestimmten Schwellenwert überschreitet, ob eine Änderung in einer Grenze der ROI, die auf Basis der Position mit der Helligkeitsdifferenz berechnet wurde, einen vorbestimmten Schwellenwert überschreitet, ob die Anzahl vorbestimmter Normalenlinien der Grenze der ROI, die auf Basis der Position mit der Helligkeitsdifferenz berechnet wurde, einen vorbestimmten Schwellenwert überschreitet, ob ein Winkel einer vorbestimmten Normalenlinie der Grenze der ROI, die auf Basis der Position mit der Helligkeitsdifferenz berechnet wurde, einen vorbestimmten Schwellenwert überschreitet, ob eine Statistik der vorbestimmten Normalenlinien der Grenze der ROI, die auf Basis der Position mit der Helligkeitsdifferenz berechnet wurde, einen vorbestimmten Schwellenwert überschreitet, und/oder ob ein Korrelationswert mit der ROI des erfolgreichen Ultraschallbilds einen vorbestimmten Schwellenwert überschreitet, beurteilt, ob die Berechnung der ROI erfolgreich ist oder nicht.

5. Ultraschallbildgebungsgerät nach Anspruch 1, wobei der Kompensationsabschnitt auf der Grundlage, ob die Differenz zwischen der Aufnahmezeit des gescheiterten Ultraschallbilds und der Aufnahmezeit des erfolgreichen Ultraschallbilds innerhalb einer vorbestimmten Zeit liegt und/oder ob eine ID eines Diagnoseverlaufs korrespondiert, beurteilt, ob das gescheiterte Ultraschallbild mit dem erfolgreichen Ultraschallbild kompensiert werden kann.

6. Ultraschallbildgebungsgerät nach Anspruch 1, wobei
der ROI-Berechnungsabschnitt eine dreidimensionale ROI einstellt, indem kontinuierlich Grenzen zweidimensionaler ROIs von zweidimensionalen Ultraschallbildern längs einer vorbestimmten Koordinatenachse eingestellt werden,
der Beurteilungsabschnitt beurteilt, ob die Berechnung der zweidimensionalen ROIs erfolgreich ist oder nicht, und
der Kompensationsabschnitt das gescheiterte Ultraschallbild mit dem erfolgreichen Ultraschallbild kompensiert, das aus den Ultraschallbilddaten zu dem Zeitpunkt erzeugt wurde, zu dem die Berechnung der zweidimensionalen ROI erfolgreich war.

7. Ultraschallbildgebungsgerät nach Anspruch 1, wobei
der ROI-Berechnungsabschnitt eine dreidimensionale ROI einstellt, indem kontinuierlich Grenzen zweidimensionaler ROIs von zweidimensionalen Ultraschallbildern längs einer vorbestimmten Koordinatenachse angeordnet werden,
der Beurteilungsabschnitt beurteilt, ob die Berechnung der zweidimensionalen ROIs erfolgreich ist oder nicht, und
der Kompensationsabschnitt das gescheiterte Ultraschallbild mit dem erfolgreichen Ultraschallbild kompensiert, das in Echtzeit basierend auf einer Grenze der zweidimensionalen ROI zu dem Zeitpunkt, zu dem die Berechnung der zweidimensionalen ROI erfolgreich war, aus den Ultraschallbilddaten erzeugt wurde.

8. Ultraschallbildgebungsgerät nach Anspruch 1, wobei
der ROI-Berechnungsabschnitt aus den Ultraschallbilddaten eine dreidimensionale ROI berechnet,
der Beurteilungsabschnitt beurteilt, ob die Berechnung der dreidimensionalen ROI erfolgreich ist oder nicht, und
der Kompensationsabschnitt das basierend auf der dreidimensionalen ROI gescheiterte Ultraschallbild, das dreidimensional ist, mit dem basierend auf der dreidimensionalen ROI erfolgreichen Ultraschallbild kompensiert, das dreidimensional ist.

9. Ultraschallbildgebungsgerät nach Anspruch 1, wobei der ROI-Berechnungsabschnitt die Ultraschallbilddaten in mehrere Blöcke unterteilt, die mehreren Blöcke basierend auf der Helligkeit jedes Blocks klassifiziert, und eine Grenze der klassifizierten Blöcke als eine dreidimensionale ROI berechnet.

10. Ultraschallbildgebungsgerät nach Anspruch 1, wobei der Beurteilungsabschnitt die Ultraschallbilddaten in mehrere Blöcke unterteilt und basierend auf einer Position des Blocks mit einer vorbestimmten Helligkeitsdifferenz und/oder der Anzahl der Blöcke mit der Helligkeitsdifferenz beurteilt, ob die Berechnung der ROI erfolgreich ist oder nicht.

11. Ultraschallbildgebungsgerät nach Anspruch 1, mit einem Markierungserzeugungsabschnitt, der dazu konfiguriert ist, die Anzeige einer Markierung, die anzeigt, ob die Berechnung der ROI erfolgreich ist oder nicht, auf dem erfolgreichen Ultraschallbild zu veranlassen.

12. Ultraschallbildgebungsgerät nach Anspruch 11, wobei der Markierungserzeugungsabschnitt die Anzeige einer sich von den anderen erfolgreichen Ultraschallbildern unterscheidenden Markierung auf dem erfolgreichen Ultraschallbild auslöst, mit dem das gescheiterte Ultraschallbild kompensiert wurde.

13. Ultraschallbildgebungsgerät nach Anspruch 1, mit einem Anzeigeabschnitt, der dazu konfiguriert ist, auf dem erfolgreichen Ultraschallbild anzuzeigen, ob die Berechnung der ROI erfolgreich ist oder nicht.

14. Ultraschallbildgebungsgerät nach Anspruch 1, mit einem Stimmenerzeugungsabschnitt, der dazu konfiguriert ist, eine Stimme zu erzeugen, die angibt, ob die Berechnung der ROI erfolgreich ist oder nicht.

15. Ultraschallbildanzeigeverfahren, in dem
aus Ultraschallbilddaten eine ROI berechnet wird,
basierend auf einer Position mit einer vorbestimmten Helligkeitsdifferenz und/oder der Anzahl von Positionen mit der Helligkeitsdifferenz in den Ultraschallbilddaten beurteilt wird, ob die Berechnung der ROI erfolgreich ist oder nicht, und
ein basierend auf der als gescheitert beurteilten ROI gescheitertes Ultraschallbild mit einem basierend auf der als erfolgreich beurteilten ROI erfolgreichen Ultraschallbild kompensiert wird.

## Revendications

1. Dispositif d'imagerie ultrasonore comprenant :
une section de calcul de ROI pour calculer une ROI à partir de données d'image ultrasonore ;
une section de jugement configurée pour juger si le calcul de la ROI est un succès ou non en fonction d'au moins un d'une position avec une différence de luminosité prédéterminée et du nombre de positions avec la différence de luminosité dans les données d'image ultrasonore ; et
une section de compensation configurée pour compenser une image ultrasonore ratée en fonction de la ROI jugée comme ratée par la section de jugement avec une image ultrasonore réussie en fonction de la ROI jugée comme réussie par la section de jugement.

2. Dispositif d'imagerie ultrasonore selon la revendication 1, dans lequel la section de compensation compense l'image ultrasonore ratée avec l'image ultrasonore réussie générée à partir des données d'image ultrasonore au moment de la réussite du calcul de la ROI.

3. Dispositif d'imagerie ultrasonore selon la revendication 1, dans lequel la section de compensation compense l'image ultrasonore ratée avec l'image ultrasonore réussie générée en temps réel à partir des données d'image ultrasonore en fonction d'une limite de la ROI au moment de la réussite du calcul de la ROI.

4. Dispositif d'imagerie ultrasonore selon la revendication 1, dans lequel la section de jugement juge si le calcul de la ROI est un succès ou non en fonction d'au moins un de si le nombre de positions avec la différence de luminosité dépasse un seuil prédéterminé ou non, si un taux du nombre des positions avec la différence de luminosité dépasse un seuil prédéterminé ou non, si une statistique du nombre des positions avec la différence de luminosité dépasse un seuil prédéterminé ou non, si un changement des coordonnées de la ROI calculée en fonction de la position avec la différence de luminosité dépasse un seuil prédéterminé ou non, si un changement dans la taille de la ROI calculée en fonction de la position avec la différence de luminosité dépasse un seuil prédéterminé ou non, si un changement d'une limite de la ROI calculée en fonction de la position avec la différence de luminosité dépasse un seuil prédéterminé ou non, si le nombre de lignes normales prédéterminées de la limite de la ROI calculée en fonction de la position avec la différence de luminosité dépasse un seuil prédéterminé ou non, si un angle d'une ligne normale prédéterminée de la limite de la ROI calculée en fonction de la position avec la différence de luminosité dépasse un seuil prédéterminé ou non, si une statistique des lignes normales prédéterminées de la limite de la ROI calculée en fonction de la position avec la différence de luminosité dépasse un seuil prédéterminé ou non, et si une valeur de corrélation avec la ROI de l'image ultrasonore réussie dépasse un seuil prédéterminé ou non.

5. Dispositif d'imagerie ultrasonore selon la revendication 1, dans lequel la section de compensation juge si l'image ultrasonore ratée peut être compensée avec l'image ultrasonore réussie en fonction d'au moins un de si la différence entre le temps d'acquisition de l'image ultrasonore ratée et le temps d'acquisition de l'image ultrasonore réussie est dans un temps prédéterminé ou non et de si une ID d'un tableau de diagnostic correspond ou non.

6. Dispositif d'imagerie ultrasonore selon la revendication 1, dans lequel
la section de calcul de ROI règle une ROI tridimensionnelle en agençant de manière continue des limites des ROI bidimensionnelles d'images ultrasonores bidimensionnelles le long d'un axe de coordonnées prédéterminé ;
la section de jugement juge si le calcul des ROI bidimensionnelles est un succès ou non ; et
la section de compensation compense l'image ultrasonore ratée avec l'image ultrasonore réussie générée à partir des données d'image ultrasonore au moment de la réussite du calcul de la ROI bidimensionnelle.

7. Dispositif d'imagerie ultrasonore selon la revendication 1, dans lequel
la section de calcul de ROI règle une ROI tridimensionnelle en agençant de manière continue des limites des ROI bidimensionnelles des images ultrasonores bidimensionnelles le long d'un axe de coordonnées prédéterminé ;
la section de jugement juge si le calcul des ROI bidimensionnelles est un succès ou non ; et
la section de compensation compense l'image ultrasonore ratée avec l'image ultrasonore réussie générée en temps réel à partir de données d'image ultrasonore en fonction d'une limite de la ROI bidimensionnelle au moment de la réussite du calcul de la ROI bidimensionnelle.

8. Dispositif d'imagerie ultrasonore selon la revendication 1, dans lequel
la section de calcul de ROI calcule une ROI tridimensionnelle à partir des données d'image ultrasonore ;
la section de jugement juge si le calcul de la ROI tridimensionnelle est un succès ou non ; et
la section de compensation compense l'image ultrasonore ratée qui est tridimensionnelle, en fonction de la ROI tridimensionnelle avec l'image ultrasonore réussie, qui est tridimensionnelle, en fonction de la ROI tridimensionnelle.

9. Dispositif d'imagerie ultrasonore selon la revendication 1, dans lequel la section de calcul de ROI divise les données d'image ultrasonore en une pluralité de blocs, classifie la pluralité de blocs en fonction de la luminosité de chaque bloc, et calcule une limite du bloc classifié comme une ROI tridimensionnelle.

10. Dispositif d'imagerie ultrasonore selon la revendication 1, dans lequel la section de jugement divise les données d'image ultrasonore en une pluralité de blocs et juge si le calcul de la ROI est un succès ou non en fonction d'au moins un d'une position du bloc ayant une différence de luminosité prédéterminée et du nombre des blocs ayant la différence de luminosité.

11. Dispositif d'imagerie ultrasonore selon la revendication 1, comprenant une section de génération de marques configurée pour faire s'afficher une marque montrant si le calcul de la ROI est un succès ou non sur l'image ultrasonore réussie.

12. Dispositif d'imagerie ultrasonore selon la revendication 11, dans lequel la section de génération de marques fait s'afficher une marque différente des autres images ultrasonores réussies sur l'image ultrasonore réussie avec laquelle l'image ultrasonore ratée est compensée.

13. Dispositif d'imagerie ultrasonore selon la revendication 1, comprenant une section d'affichage configurée pour afficher si le calcul de la ROI est un succès ou non sur l'image ultrasonore réussie.

14. Dispositif d'imagerie ultrasonore selon la revendication 1, comprenant une section de génération vocale configurée pour générer une voix montrant si le calcul de la ROI est un succès ou non.

15. Procédé d'affichage d'image ultrasonore comprenant :
de calculer une ROI à partir de données d'image ultrasonore ;
de juger si le calcul de la ROI est un succès ou non en fonction d'au moins un d'une position avec une différence de luminosité prédéterminée et du nombre de positions avec la différence de luminosité dans les données d'image ultrasonore ; et
de compenser une image ultrasonore ratée en fonction de la ROI jugée comme ratée avec une image ultrasonore réussie en fonction de la ROI jugée comme réussie.
